(19) Europäisches Patentamt
European Patent Office
Office européen des brevets

(11) EP 0 974 635 A1

(12) EUROPEAN PATENT APPLICATION

(43) Date of publication:
**26.01.2000 Bulletin 2000/04**

(51) Int. Cl.[7]: **C09K 19/20**, C07C 69/94

(21) Application number: **99113531.0**

(22) Date of filing: **06.07.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **21.07.1998 JP 20466498**

(71) Applicant:
**MITSUBISHI GAS CHEMICAL COMPANY, INC.**
**Tokyo 100-0005 (JP)**

(72) Inventors:
- **Mine, Takakiyo,**
**Corporate Research Laboratory**
**Tsukuba-shi, Ibaraki-ken 300-4247 (JP)**
- **Johno, Masahiro,**
**Corporate Research Laboratory**
**Tsukuba-shi, Ibaraki-ken 300-4247 (JP)**
- **Yui, Tomoyuki,**
**Corporate Research Laboratory**
**Tsukuba-shi, Ibaraki-ken 300-4247 (JP)**
- **Matsumoto, Takahiro,**
**Corporate Research Laboratory**
**Tsukuba-shi, Ibaraki-ken 300-4247 (JP)**

(74) Representative:
**Kraus, Walter, Dr. et al**
**Kraus & Weisert,**
**Thomas-Wimmer-Ring 15**
**80539 München (DE)**

# (54) Ferrielectric liquid crystal compound

(57) A ferrielectric liquid crystal compound represented by the following formula (1),

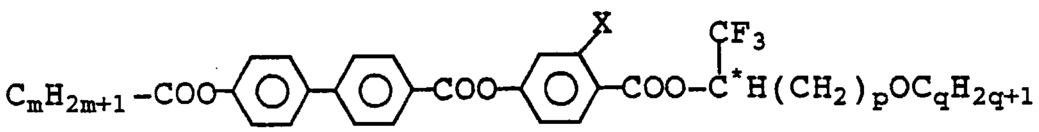

(1)

wherein m is an integer of 6 to 12, X is a hydrogen atom or a fluorine atom, p is an integer of 2 to 4, q is an integer of 2 to 4, and C* is an asymmetric carbon atom.

The above ferrielectric liquid crystal compound is very useful as a practical material for an active-matrix liquid crystal display device.

EP 0 974 635 A1

## Description

Field of the Invention

The present invention relates to a novel ferrielectric liquid crystal compound suitable for use in an active matrix-type liquid crystal display device in which a liquid crystal is driven for each pixel independently of another, and to its use.

Prior Art

A liquid crystal display device (LCD) has been being widely used as a flat panel display as a substitute for a conventional display using a Braun tube mainly in portable machines and equipment. Along with the recent functional expansion of personal computers and word processors and with the recent increase in the capacity of data processing, LCD is also required to have higher functions, that is, to have a large display capacity, a full-color display, a wide viewing angle, a high-speed response and a high contrast.

As a liquid crystal display method (liquid crystal driving method) to comply with such requirements, there is proposed and practically used an active matrix (AM) display device which works by a method in which thin film transistors (TFT) or diodes (MIM) are formed such that one transistor or diode corresponds to one pixel on a display screen and a liquid crystal is driven for one pixel independently of another.

The above display method has problems that it is difficult to decrease a cost due to a low production yield and that it is difficult to form a large-sized display screen. Due to a high display quality, however, the above display method is about to surpass an STN display method which has been so far a mainstream and to overtake CRT.

Problems to be Solved by the Invention

However, the above AM display device has the following problems due to the use of a TN (twisted nematic) liquid crystal as a liquid crystal material.

(1) A TN liquid crystal is a nematic liquid crystal, and the response speed is generally low (several tens millisecond (ms)). In the display of video rate, no good image quality can be obtained.

(2) A twisted state (twist alignment) of liquid crystal molecules is used for displaying, and the viewing angle is therefore narrow. In the display with a gray scale in particular, the viewing angle is sharply narrowed. That is, the contrast ratio, the color or the like change depending upon viewing angles to a display screen.

For overcoming the above problems, there have been proposed, in recent years, AM panels which use a ferroelectric liquid crystal or an anti-ferroelectric liquid crystal in place of the TN liquid crystal (Japanese Laid-open Patent Publications Nos. 5-249502, 5-150257 and 6-95080). However, at present, the following problems remain to solve for the practical use of these liquid crystals.

(3) A ferroelectric liquid crystal has spontaneous polarization. An image sticking is liable to occur due to the constant presence of the spontaneous polarization and the driving is hence made difficult. In the display with a ferroelectric liquid crystal, it is very difficult to perform a gray-scale display since only a binary state of black and white is possible in principle.

For the gray-scale display, special devising is required (for example, use of a ferroelectric liquid crystal device using monostability; Keiichi NITO et al., SID '94, Preprint, p. 48), and it is required to develop a high technique for practical use.

(4) An anti-ferroelectric liquid crystal is free from the image sticking problem described in the above (3) since it has no permanent spontaneous polarization.

However, in the AM driving, there is needed a liquid crystal material which can be at least driven at 10 V or less. But, the anti-ferroelectric liquid crystal generally shows a high threshold voltage, and its driving at a low voltage is therefore difficult. Further, it has another problem that the gray-scale display is difficult to perform since its optical response involves a hysteresis.

An object of the present invention aims at providing a novel material which can overcome the above problems and is suitable for use in AM driving, and a ferrielectric liquid crystal is thinkable as the above novel material.

A ferrielectric liquid crystal having a ferrielectric phase (Scγ* phase) was found for the first time in 4-(1-methylheptyloxycarbonyl)phenyl-4-(4'-octyloxybiphenyl)carboxylate (called "MHPOBC" for short) in 1989 (Japanese Journal of Applied Physics, Vol. 29, No. 1, 1990, pp. L131 - 137).

The chemical structural formula and phase transition temperatures (°C) of the MHPOBC are as follows.

Structural formula:

$$C_8H_{17}\text{-O-Ph-Ph-COO-Ph-COO-C*H}(CH_3)\text{-}C_6H_{13}$$

wherein Ph is a 1,4-phenylne group and C* is an asymmetric carbon atom.

Phase sequence:

Cr(30)SIA*(65)SCA*(118)SCγ*(119)SC*(121)SCα*(122)SA(147)I

wherein Cr is a crystal phase, SIA* is a chiral smectic IA phase, SCA* is a chiral smectic CA phase (anti-ferroelectric phase), SCγ* is a chiral smectic Cγ phase (ferrielectric phase), SC* is a chiral smectic C phase (ferroelectric phase), SCα* is a chiral smectic Cα phase, SA is a smectic A phase, and I is an isotropic phase.

Brief Description of Drawings

Fig. 1 shows a molecular arrangement states of a ferrielectric phase. FI(+) and FI(-) show a ferrielectric state, and FO(+) and FO(-) show an ferroelectric state.
Fig. 2 shows an optical response of a ferrielectric phase to a triangular wave voltage.

The ferrielectric liquid crystal will be explained by referring to the drawings.
To explain a ferrielectric liquid crystal, Fig. 1 shows molecular arrangement states of a ferrielectric phase, and Fig. 2 shows an optical response of a ferrielectric phase to a triangular wave.
A ferrielectric phase has a molecular arrangement of FI(+) (a case where an applied voltage is positive) or a molecular arrangement of FI(-) (a case where an applied voltage is negative) as shown in Fig. 1.
In a state free of an electric field, FI(+) and FI(-) are equivalent and are therefore co-present.
In the state free of an electric field, therefore, average optic axis is in the direction of a layer normal, and the state is in a dark state under the condition of a polarizer shown in Fig. 1. This state corresponds to a part showing a voltage of 0 in Fig. 2.
Further, although each of FI(+) and FI(-) has spontaneous polarization as is apparent from the molecular arrangement states, the spontaneous polarizations are cancelled in a state in which these are co-present, and an average spontaneous polarization is consequently zero. This shows that, like an anti-ferroelectric liquid crystal, a ferrielectric liquid crystal is free from an image sticking phenomenon observed in a ferroelectric liquid crystal.
As an electric field is applied to a ferrielectric phase, a region (domain) having an extinction position appears at a voltage lower than a voltage at which a ferroelectric phase is reached. This shows that the above domain has an optic axis in the direction that tilts from the direction of layer normal although the tilt is not so large as that in a ferroelectric state.
The above intermediate state is considered to be FI(+) or FI(-).
In the present invention, a liquid crystal phase which always shows at least the above intermediate state is called a ferrielectric phase, and if a liquid crystal compound has the broader ferrielectric phase in its phase sequence compared with another phase is called a ferrielectric liquid crystal compound.
When the applied voltage is further increased, the ferrielectric phase causes a phase transition to a ferroelectric phase FO(+) or FO(-) that is a stabilized state, depending upon a direction of the electric field. In Fig. 2, a phase in which the intensity of transmitted-light is brought into a saturated state (flat parts on left and right sides) is FO(+) or FO(-).
In the above ferroelectric state FO(+) or FO(-), there is exhibited a spontaneous polarization greater than that in the ferrielectric phase FI(+) or FI(-), as is seen in Fig. 1. The response speed increases with an increase in the spontaneous polarization, and as a result, the capability of high speed response is materialized.
Both the ferroelectric states are in a light state under the condition of a polarizer shown in Fig. 1.
A conventional ferroelectric liquid crystal provides a switching between FO(+) and FO(-), while the ferrielectric liquid crystal has a great characteristic feature that it permits switching among four states of FO(+), FI(+), FI(-) and FO(-).
In the ferrielectric phase, therefore, not a continuous change in the intensity of transmitted light between voltages of 0 V and 4 V but a stepwise change in the intensity of transmitted light ought to be observed.
In Fig. 2, however, a continuous change in the intensity of transmitted light is observed.

It is assumed that the above occurs because the threshold voltage from the co-presence state of FI(+) and FI(-) to FO(+) via FI(+) or the threshold voltage from the co-presence state of FI(+) and FI(-) to FO(-) via FI(-) is not clear.

As shown in Fig. 2, generally, a ferrielectric liquid crystal has a tendency that a difference between the voltage required for change from a ferrielectric state to a ferroelectric state and the voltage required for change from a ferroelectric state to a ferrielectric state is small. That is, the width of its hysteresis is very narrow. It characteristically shows a V-letter-shaped optical response and therefore has properties suitable for AM driving and a display with a gray scale in AM driving.

Further, in the change of the ferrielectric liquid crystal by a voltage, the applied voltage (phase transition voltage) required for a change from a ferrielectric state to a ferroelectric state tends to be very small as compared with that of an anti-ferroelectric liquid crystal, and it can be therefore said that the ferrielectric liquid crystal is suitable for AM driving.

In the ferrielectric phase, generally, the change between the co-presence state of FI(+) and FI(-) and a ferroelectric state (FO(+) or FO(-)) is continuous as shown in Fig. 2, and the voltage required for the change is small. Further, the light transmittance in the co-presence state of FI(+) and FI(-) at an applied voltage of 0 can be further decreased by devising an alignment film.

On the basis of these, in the ferrielectric liquid crystal, the co-presence state of FI(+) and FI(-) can be used as dark, the ferroelectric states FO(+) and FO(-) as light and an intermediate state of these as gray. The display principle thereof uses birefringence of a liquid crystal, and a display device having a decreased viewing angle dependency can be produced as the liquid crystal molecules are arranged in parallel with the glass substrate.

However, the number of ferrielectric liquid crystals that have been synthesized so far is very small, and when application to an AM driving device is taken into account, few ferrielectric liquid crystals that have been already known are satisfactory in respect of hysteresis and a voltage in the transition from a ferrielectric phase to a ferroelectric phase (phase transition voltage).

Means to Solve the Problems

Under the circumstances, the present inventors have made studies to find a novel ferrielectric liquid crystal compound having a narrow width of hysteresis, a small phase transition voltage and excellent characteristic properties as AM driving device, and as a result, have arrived at the present invention.

That is, according to the present invention, there is provided a ferrielectric liquid crystal compound of the following formula (1),

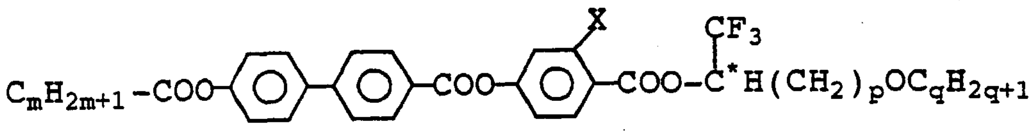 (1)

wherein m is an integer of 6 to 12, X is a hydrogen atom or a fluorine atom, p is an integer of 2 to 4, q is an integer of 2 to 4, and C* is an asymmetric carbon atom.

In the ferrielectric liquid crystal compound of the above formula (1) in the present invention, m is an integer of 6 to 12, preferably an integer of 9 to 12. And, X is a hydrogen or fluorine atom, p is an integer of 2 to 4 and q is an integer of 2 to 4, preferably 2. The ferrielectric liquid crystal compound of the general formula (1) in which m is 9 to 12 and q is 2 is excellent from the viewpoints of the voltage for a transition from a ferrielectric state to a ferroelectric state, a temperature range of the ferrielectric phase and the spontaneous polarization.

When the ferrielectric liquid crystal compound of the present invention is considered as a raw material for practical use, the transition temperature (transition temperature on the high-temperature side) for the transition from an isotropic phase, a smectic A phase or a chiral smectic C phase to a ferrielectric phase is preferably at least 40°C. On the other hand, the temperature range of the ferrielectric phase is preferably at least 10°C from the practical standpoint.

The ferrielectric liquid crystal provided by the present invention can form an active matrix liquid crystal display device when interposed between substrates on which non-linear active devices such as thin film transistors or diodes are provided for individual pixels.

The ferrielectric liquid crystal compound of the invention can switch to a co-presence state of two ferrielectric phases, to two ferroelectric phases and to an intermediate state between them by changing the voltage. Further, a difference between a voltage for change from the ferrielectric state to the ferroelectric state and a voltage for change from the ferroelectric state to the ferrielectric state is small, and it is preferably 0.5 V or lower.

An optically active alcohol $CF_3C^*H(OH)(CH_2)_pOC_qH_{2q+1}$ used for the synthesis of the compound of the present invention can be easily produced by the method that the present inventors have already found.

The method of the production thereof, for example, when p is 3 and q is 2, is outlined as follows.

(a)

$$BrCH_2CH_2CH_2Br + NaOC_2H_5 \rightarrow Br(CH_2)_3OC_2H_5$$

(b)

$$Br(CH_2)_3OC_2H_5 + Mg \rightarrow MgBr(CH_2)_3OC_2H_5$$

(c)

$$MgBr(CH_2)_3OC_2H_5 + CF_3COOH \rightarrow CF_3CO(CH_2)_3OC_2H_5$$

(d)

$$CF_3CO(CH_2)_3OC_2H_5 + LiAlH_4 \rightarrow CF_3CH(OH)(CH_2)_3OC_2H_5$$

(e)

$$CF_3CH(OH)(CH_2)_3OC_2H_5 + CH_3COCl \rightarrow CF_3CH(OCOCH_3)(CH_2)_3OC_2H_5$$

(f)

$$CF_3CH(OCOCH_3)(CH_2)_3OC_2H_5 + \text{(lipase)} \rightarrow \text{R-(+)-}CF_3C^*H(OH)(CH_2)_3OC_2H_5 + \text{S-(-)-}$$

$$CF_3C^*H(OCOCH_3)(CH_2)_3OC_2H_5$$

The above method of the production of the optically active alcohol will be briefly explained as follows.

(a) 1,3-propane dibromide is converted to an ethoxy compound.
(b) A Grignard reagent is prepared.
(c) A ketone is prepared by reacting the Grignard reagent and trifluoroacetic acid.
(d) An alcohol is prepared by subjecting the ketone (c) to hydrogenation.
(e) The alcohol (d) is acetylated
(f) Acetate (e) is asymmetrically hydrolyzed with lipase, whereby an R-form optically active alcohol as an end product and an S-form acetate are obtained.

Effect of the Invention

The novel ferrielectric liquid crystal compound provided by the present invention has a ferrielectric phase in a broad temperature range, and is very useful as a practical raw material.

Examples

The present invention will be explained in more detail with reference to Examples hereinafter, while the present invention shall not be limited thereto.

<u>Example 1</u> (formula (1): m = 10, X = F, p = 3, q = 2 (E1))

Preparation of R-(+)-3-fluoro-4-(1-trifluoromethyl-4-ethoxybutyloxycarbonyl)phenyl-4'-n-undecanoyloxybiphenyl-4-carboxylate

(1) Preparation of 4-(4'-n-undecanoyloxy)biphenylcarboxylic acid

10.0 Grams of 4-(4'-hydroxy)biphenylcarboxylic acid, 9.7 g of n-undecanoic acid chloride, 16 ml (milliliter) of triethyl amine and 1 g of dimethylaminopyridine were dissolved in 150 ml dichloromethane and the mixture was stirred

at room temperature for one day and night.

After completion of the reaction, the reaction mixture was extracted with 100 ml of ether three times after addition of 50 ml of a 10 % hydrochloric acid. The organic phase was washed with 100 ml of a saline solution three times and dried over anhydrous sodium sulfate.

After distilling off the solvent, the distillate was washed with 400 ml of hexane to obtain an end product.

(2) Preparation of 4-acetoxy-2-fluorobenzoic acid

4.3 Grams of 2-fluoro-4-hydroxybenzoic acid and 8.4 g of acetic anhydride were placed in a two-necked flask and mixed. While the mixture was cooled with water, 5 drops of sulfuric acid were added. After heat generation ended, the mixture was heated at 80°C for 30 minutes. Thereafter, the reaction mixture was poured into cold water, and a precipitated crystal was recovered by filtration.

The crystal was dried in vacuum, and used in the next step.

(3) Preparation of R-(+)-4-acetoxy-2-fluoro-1-(1-trifluoromethyl-4-ethoxybutyloxycarbonyl)benzene

1.0 Gram of 4-acetoxy-2-fluorobenzoic acid was added to 7 ml of thionyl chloride, and the mixture was allowed to react under reflux for 5 hours. Then, excessive thionyl chloride was distilled off, and a mixture containing 1 ml of pyridine, 4 ml of dry ether and 0.6 g of R-(+)-1,1,1-trifluoro-5-ethoxypentan-2-ol was added dropwise.

After the addition, the mixture was stirred at room temperature for one day and night, and diluted with 200 ml of ether, and an organic layer was washed with diluted hydrochloric acid, with a 1N sodium hydroxide aqueous solution and with water in this order, and then dried over magnesium sulfate. The solvent was distilled off, and the resultant crude product was purified by silica gel column chromatography using hexane/ethyl acetate as a solvent, to give an end product.

(4) Preparation of R-(+)-4-hydroxy-2-fluoro-1-(1-trifluoromethyl-4-ethoxybutyloxycarbonyl)benzene

1.0 Gram of the compound obtained in the above (3) was dissolved in 30 ml of ethanol, and 3 g of benzylamine was added dropwise thereto. Further, the mixture was stirred at room temperature for one day and night, diluted with 300 ml of ether, washed with diluted hydrochloric acid and then with water, and dried over magnesium sulfate.

The solvent was distilled off, and the remainder was subjected to silica gel column chromatography for isolation and purification to give an end product.

(5) Preparation of R-(+)-3-fluoro-4-(1-trifluoromethyl-4-ethoxybutyloxycarbonyl)phenyl-4'-n-undecanoyloxybiphenyl-4-carboxylate

To 1.0 g of the compound obtained in the above (1) was added to 10 ml of thionyl chloride, and the mixture was refluxed under heating for 10 hours. Excessive thionyl chloride was distilled off, and 10 ml of pyridine and 25 ml of toluene were added to the mixture. Then, 25 ml of a benzene solution containing 0.8 g of the compound obtained in the above (4) was added dropwise, and the mixture was allowed to react at room temperature for 10 hours.

After completion of the reaction, the reaction mixture was diluted with 300 ml of ether and washed with diluted hydrochloric acid, with a 1N sodium carbonate aqueous solution, and with water in this order, and an organic layer was dried over magnesium sulfate. Then, the solvent was distilled off, the remainder was isolated by silica gel column chromatography, and ethanol was used for recrystallization, to give an end product.

<u>Example 2</u> (formula (1): m = 9, X = H, p = 3, q = 2 (E2)), Preparation of R-(+)-4-(1-trifluoromethyl-4-ethoxybutyloxycarbonyl)phenyl-4'-n-decanoyloxybiphenyl-4-carboxylate

End product was obtained in the same manner as in Example 1 except that the n-undecanoic acid chloride in the (1) of Example 1 was replaced with n-decanoic acid chloride and that the 2-fluoro-4-hydroxybenzoic acid in the (2) of Example 1 was replaced with 4-hydroxybenzoic acid.

<u>Example 3</u> (formula (1): m = 10, X = H, p = 3, q = 2 (E3)), Preparation of R-(+)-4-(1-trifluoromethyl-4-ethoxybutyloxycarbonyl)phenyl-4'-n-undecanoyloxybiphenyl-4-carboxylate

An end product was obtained in the same manner as in Example 1 except that the 2-fluoro-4-hydroxybenzoic acid in the (2) of Example 1 was replaced with 4-hydroxybenzoic acid.

Example 4 (formula (1): m = 12, X = H, p = 3, q = 2 (E4)), Preparation of R-(+)-4-(1-trifluoromethyl-4-ethoxybutyloxycarbonyl)phenyl-4'-n-tridecanoyloxybiphenyl-4-carboxylate

An end product was obtained in the same manner as in Example 1 except that the n-undecanoic acid chloride in the (1) of Example 1 was replaced with n-tridecanoic acid chloride and that the 2-fluoro-4-hydroxybenzoic acid in the (2) of Example 1 was replaced with 4-hydroxybenzoic acid.

Example 5 (formula (1): m = 9, X = F, p = 2, q = 2 (E5)), Preparation of R-(+)-3-fluoro-4-(1-trifluoromethyl-3-ethoxypropyloxycarbonyl)phenyl-4'-n-decanoyloxybiphenyl-4-carboxylate

An end product was obtained in the same manner as in Example 1 except that the n-undecanoic acid chloride in the (1) of Example 1 was replaced with n-decanoic acid chloride and that the R-(+)-1,1,1-trifluoro-5-ethoxypentan-2-ol in the (3) of Example 1 was replaced with R-(+)-1,1,1-trifluoro-4-ethoxybutan-2-ol.

Example 6 (formula (1): m = 9, X = F, p = 4, q = 2 (E6)), Preparation of R-(+)-3-fluoro-4-(1-trifluoroxnethyl-3-ethoxypentyloxycarbonyl)phenyl-4'-n-decanoyloxybiphenyl-4-carboxylate

An end product was obtained in the same manner as in Example 1 except that the n-undecanoic acid chloride in the (1) of Example 1 was replaced with n-decanoic acid chloride and that the R-(+)-1,1,1-trifluoro-5-ethoxypentan-2-ol in the (3) of Example 1 was replaced with R-(+)-1,1,1-trifluoro-4-ethoxyhexan-2-ol.

Table 1 shows $^1$H-NMR spectrum data of the end products obtained in the above Examples 1 to 6.

Liquid crystal phases were identified as follows, and the results are shown in Table 2. The liquid crystal compounds were identified for liquid crystal phases by texture observation, conoscopic image observation and DSC (differential scanning calorimeter) measurement. The observation of a conoscopic image is an effective means for identifying a ferrielectric phase. The conoscopic image observation was conducted according to a literature (J. Appl. Phys. 31, 793 (1992)).

The texture observation based on general parallel alignment cells, the conoscopic observation and the DSC measurement showed that the end products of the present invention were ferrielectric liquid crystals.

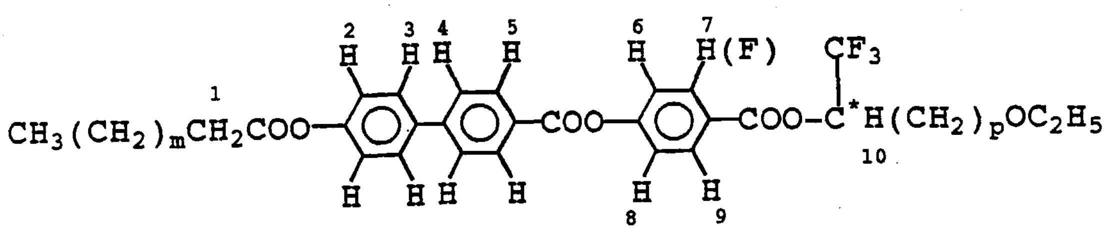

Table 1

| | Chemical shift (ppm) | | | | | | | | | |
|---|---|---|---|---|---|---|---|---|---|---|
| | 1H | 2H | 3H | 4H | 5H | 6H | 7H | 8H | 9H | 10H |
| Example 1 (E1) | 2.6 | 7.2 | 7.6 | 7.8 | 8.3 | 7.1 | | 7.1 | 8.1 | 5.6 |
| Example 2 (E2) | 2.6 | 7.2 | 7.6 | 7.8 | 8.3 | 7.3 | 8.2 | 7.3 | 8.2 | 5.6 |
| Example 3 (E3) | 2.6 | 7.2 | 7.6 | 7.8 | 8.3 | 7.3 | 8.2 | 7.3 | 8.2 | 5.6 |
| Example 4 (E4) | 2.6 | 7.2 | 7.6 | 7.8 | 8.3 | 7.4 | 8.2 | 7.4 | 8.2 | 5.6 |
| Example 5 (E5) | 2.6 | 7.2 | 7.6 | 7.7 | 8.3 | 7.2 | | 7.1 | 8.2 | 5.8 |
| Example 6 (E6) | 2.6 | 7.2 | 7.6 | 7.7 | 8.3 | 7.1 | | 7.1 | 8.1 | 5.6 |

Table 2

| | Phase sequence |
|---|---|
| Example 1 (E1) | Cr(48)SCγ*(101)SA(105)I |
| Example 2 (E2) | Cr(32)SCγ*(98)SA(107)I |
| Example 3 (E3) | Cr(49)SCγ*(102)SA(115)I |
| Example 4 (E4) | Cr(79)SCγ*(97)SA(109)I |
| Example 5 (E5) | Cr(53)SCγ*(92)SA(102)I |
| Example 6 (E6) | Cr(73)SCγ*(97)SA(104)I |

In the phase sequence, parenthesized values show phase transition temperatures (°C), Cr is a crystal phase, SCγ* is a ferrielectric phase, SA is a smectic A phase, and I is an isotropic phase.

**Claims**

1. A ferrielectric liquid crystal compound represented by the following formula (1),

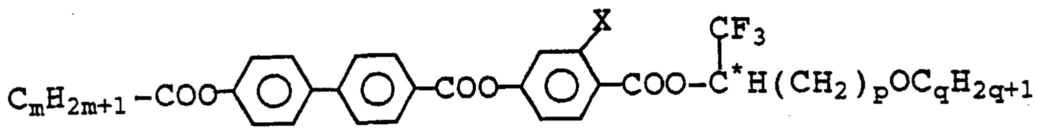

(1)

wherein m is an integer of 6 to 12, X is a hydrogen atom or a fluorine atom, p is an integer of 2 to 4, q is an integer of 2 to 4, and C* is an asymmetric carbon atom.

2. The ferrielectric liquid crystal compound of claim 1, wherein in the formula (1), m is an integer of 9 to 12.

3. The ferrielectric liquid crystal compound of claim 1, wherein in the formula (1), q is 2.

4. The ferrielectric liquid crystal compound of claim 1, wherein a transition temperature of a ferrielectric phase on a high-temperature side is at least 40°C.

5. The ferrielectric liquid crystal compound of claim 1, wherein a difference between a transition temperature of a ferrielectric phase on a high-temperature side and a transition temperature of the ferrielectric phase on a low-temperature side is at least 10°C.

6. The ferrielectric liquid crystal compound of claim 1, which has functions to switch to a co-presence state of two ferrielectric phases, to two ferroelectric phases and to an intermediate state between them by changing the voltage.

7. The ferrielectric liquid crystal compound of claim 1, wherein a difference between a voltage for a change from a ferrielectric state to a ferroelectric state and a voltage for a change from a ferroelectric state to a ferrielectric state is small.

8. An active-matrix liquid crystal display device, in which the ferrielectric liquid crystal of claim 1 is interposed between substrates on which non-linear active devices of thin film transistors or diodes are provided for individual pixels.

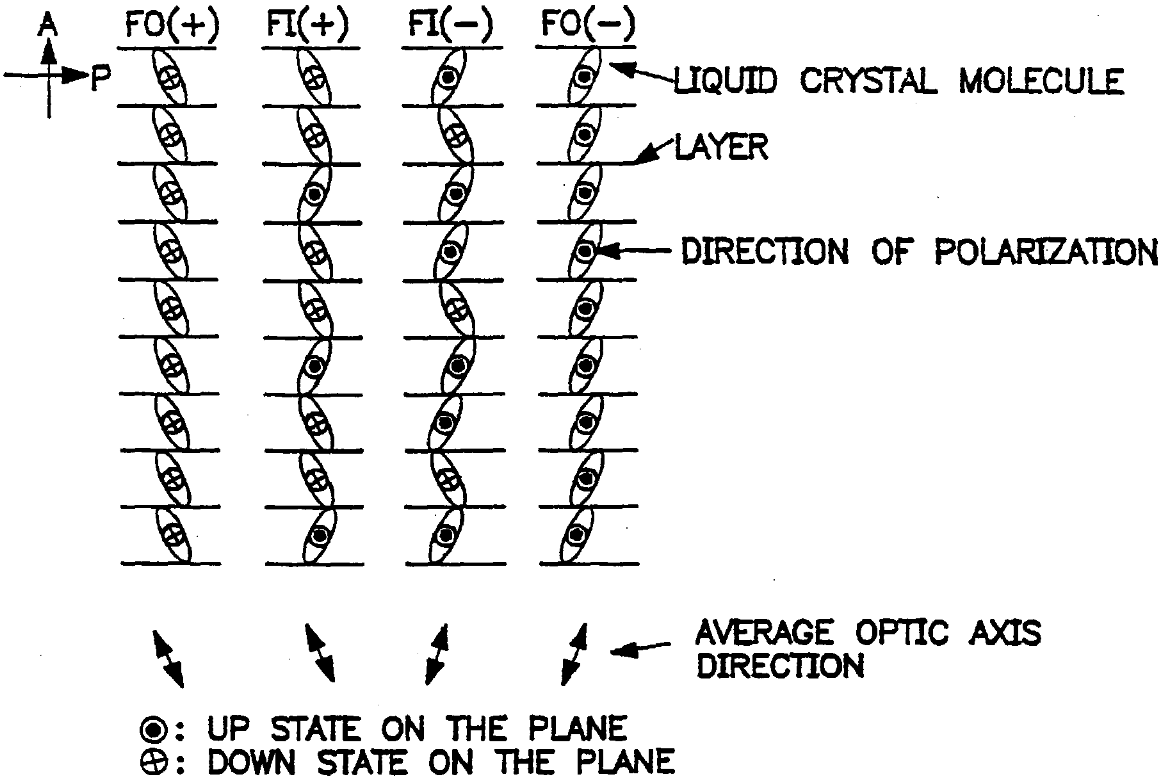
A
P
FO(+)
FI(+)
FI(−)
FO(−)
LIQUID CRYSTAL MOLECULE
LAYER
DIRECTION OF POLARIZATION
AVERAGE OPTIC AXIS DIRECTION
◉: UP STATE ON THE PLANE
⊕: DOWN STATE ON THE PLANE

FIG. 1

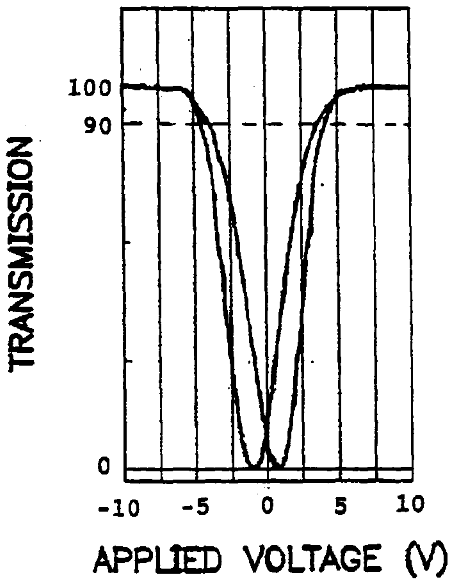
100
90
0
TRANSMISSION
-10
-5
0
5
10
APPLIED VOLTAGE (V)

FIG. 2

European Patent Office

# EUROPEAN SEARCH REPORT

Application Number
EP 99 11 3531

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | CHEMICAL ABSTRACTS, vol. 130, no. 7, 15 February 1999 (1999-02-15) Columbus, Ohio, US; abstract no. 88515, MINE, TAKAKIYO ET AL: "Ferri-dielectric liquid crystal compound" XP002119061 * abstract * -& PATENT ABSTRACTS OF JAPAN vol. 199, no. 903, 31 March 1999 (1999-03-31) & JP 10 316626 A (MISUBISHI GAS CHEM) * abstract * & JP 10 316626 A (MITSUBISHI GAS CHEMICAL CO., LTD., JAPAN) 2 December 1998 (1998-12-02) --- | 1-8 | C09K19/20 C07C69/94 |
| A | EP 0 737 733 A (MITSUBISHI GAS CHEMICAL CO) 16 October 1996 (1996-10-16) * claims 1,9,21 * --- | 1-4,8 | |
| A | DE 196 53 581 A (NIPPON SOKEN) 3 July 1997 (1997-07-03) * claims 1,3 * ----- | 1 | TECHNICAL FIELDS SEARCHED (Int.Cl.7) C09K C07C |

The present search report has been drawn up for all claims

| Place of search | Date of completion of the search | Examiner |
|---|---|---|
| THE HAGUE | 15 October 1999 | Puetz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 11 3531

This annex lists the patent family members relating to the patent documents cited in the above-mentioned European search report.
The members are as contained in the European Patent Office EDP file on
The European Patent Office is in no way liable for these particulars which are merely given for the purpose of information.

15-10-1999

| Patent document cited in search report | Publication date | Patent family member(s) | Publication date |
|---|---|---|---|
| EP 0737733 A | 16-10-1996 | JP 8337555 A | 24-12-1996 |
| | | US 5728864 A | 17-03-1998 |
| | | JP 9040960 A | 10-02-1997 |
| DE 19653581 A | 03-07-1997 | JP 9183976 A | 15-07-1997 |
| | | US 5716545 A | 10-02-1998 |

EPO FORM P0459

For more details about this annex : see Official Journal of the European Patent Office, No. 12/82